# EUROPEAN PATENT SPECIFICATION

(11) **EP 3 437 481 B1**
(45) Date of publication and mention of the grant of the patent: **22.09.2021**
(21) Application number: 17775192.2
(22) Date of filing: 29.03.2017
(51) Int. Cl.: A23C 9/12, A23C 9/13, A23L 29/212, A23L 29/00, C12N 9/78, C12N 9/80, A23C 9/137, A23L 33/20

(54) **YOGURT PRODUCTION METHOD**
VERFAHREN ZUR HERSTELLUNG VON JOGHURT
PROCÉDÉ DE PRODUCTION DE YAOURT

(30) Priority: 30.03.2016 JP 2016069184
(43) Date of publication of application: 06.02.2019
(73) Proprietor: AJINOMOTO CO., INC., Chuo-ku Tokyo 104-8315 (JP); Amano Enzyme Inc., Nagoya-shi Aichi 460-8630 (JP)
(72) Inventor: SATO, Hiroaki, Kawasaki-shi Kanagawa 210-8681 (JP)
(74) Representative: Strehl Schübel-Hopf & Partner
(86) International application number: PCT/JP2017/012838
(87) International publication number: WO 2017/170657

(56) References cited:
- WO-A1-2011/024994
- US-A1- 2010 151 081
- C. LOBATO-CALLEROS ET AL: "Impact of native and chemically modified starches addition as fat replacers in the viscoelasticity of reduced-fat stirred yogurt", JOURNAL OF FOOD ENGINEERING, vol. 131, June 2014 (2014-06), pages 110-115, XP55428690, GB ISSN: 0260-8774, DOI: 10.1016/j.jfoodeng.2014.01.019
- MIWA NORIKO ET AL: "Effect of enzymatic deamidation by protein-glutaminase on the textural and microstructural properties of set yoghurt", INTERNATIONAL DAIRY JOURNAL, ELSEVIER APPLIED SCIENCE, BARKING, GB, vol. 36, no. 1, 13 December 2013 (2013-12-13), pages 1-5, XP028628845, ISSN: 0958-6946, DOI: 10.1016/J.IDAIRYJ.2013.12.002
- LOBATO-CALLEROS C. et al.: "Impact of native and chemically modified starches addition as fat replacers in the viscoelasticity of reduced-fat stirred yogurt", J. Food Eng., vol. 131, 2014, pages 110-115, XP055428690,
- MIWA N. et al.: "Effect of enzymatic deamination by protein- glutaminase on the textural and microstructural properties of set yoghurt", Int. Dairy J., vol. 36, 2014, pages 1-5, XP028628845,
- ALCAZAR-ALAY S.C. et al.: "Physicochemical properties, modifications and applications of starches from different botanical sources", Food Sci. Technol., vol. 35, no. 2, 2015, pages 215-236, XP055428693,

## Description

### [Technical Field]

The present invention relates to a production method of yogurt, comprising adding a particular enzyme and starch.

### [Background Art]

In view of the growing interest in health in recent years, ingestion of fermented milk products such as yogurt and the like having various functions is increasing. On the other hand, preference of consumers also diversifies, and improvements in not only the functionality but also taste and flavor of yogurt are required. Under such circumstances, for example, a method for producing cheese and yogurt having a smooth mouthfeel and suppressed sour taste and bitter taste by adding a protein deamidase to raw milk is known (patent document 1).

In addition, the number of consumers who care about the calorie of food increases, and also in the yogurt market, the demand for low-calorie yogurt such as low-fat yogurt, nonfat yogurt and the like is increasing. However, many of the low-fat or nonfat yogurts are very light, watery and lack body taste. To solve such problems, a method for improving smoothness to a level similar to that of conventional yogurt by using a protein deamidase is known (patent document 2).

In addition, the development of low-protein yogurt is desired for healthy individual and patients with particular diseases seeking a low-protein as well as low-fat diet. However, since low-fat or low-protein yogurt has a small solid content, syneresis increases during preservation, thus causing a problem of shape retainability. To solve such problem, naturally occurring agar and gelatin widely used in yogurt, and gums such as carrageenan, xanthan gum, and the like may be used in some cases. However, it is known that the properties peculiar to gums affect the mouthfeel and prevent natural feeling.

### [Document List]

### [Patent documents]

patent document 1: JP-B-4711464
patent document 2: JP-B-5627022
   C. Lobato-Calleros et al.:"Impact of native and chemically modified starches addition as fat replacers in the viscoelasticity of reduced-fat stirred yogurt", JOURNAL OF FOOD ENGINEERING, vol. 131, June 2014 (2014-06-01), pages 110-115, discloses the advantages of using native starches as additives in reduced fat yogurt. Miwa Noriko et al.:"Effect of enzymatic deamidation by protein-glutaminase on the textural and microstructural properties of set yogurt", INTERNATIONAL DAIRY JOURNAL, ELSEVIER APPLIED SCIENCE, BARKING, GB, vol. 36, no. 1, 13 December 2013 (2013-12-13), pages 1-5, discloses the advantageous use of protein-glutaminase in nonfat and low-fat yogurt.

### [SUMMARY OF THE INVENTION]

### [Problems to be Solved by the Invention]

The present invention aims to provide a production method of low-fat and low-protein yogurt with smooth taste, suppressed syneresis and superior shape retainability.

### [Means of Solving the Problems]

The present inventors have conducted intensive studies in an attempt to achieve the aforementioned object and found that low-fat and low-protein smooth yogurt with good taste, suppressed syneresis and superior shape retainability can be obtained by adding protein glutaminase and starch, which resulted in the completion of the present invention.

Accordingly, the present invention provides the following.
[1] A method of producing yogurt comprising a step of adding protein glutaminase to raw milk and a step of adding starch to raw milk, wherein the raw milk has a fat content of milk of not more than 1.5 wt% and a protein content of not more than 5.5 wt%.
[2] The method of [1], wherein the starch has an average particle size of not less than 1 µm and less than 40 µm.
[3] The method of [1] or [2], wherein the amount of the starch to be added is not less than 0.1 wt% and not more than 10.0 wt% relative to the raw milk.
[4] The method of any of [1] to [3], wherein the amount of protein glutaminase to be added is not less than 0.05 unit and not more than 100 units per 1 g in weight of the milk protein in the raw milk.
[5] The method of any of [1] to [4], wherein the step of adding protein glutaminase is performed before a fermentation step.
[6] The method of any of [1] to [5], wherein the step of adding starch is performed before the fermentation step.

### [Effect of the Invention]

According to the present invention, yogurt with smooth taste like yogurt made from whole milk and with low calorie can be provided.

According to the present invention, low-fat and low-protein yogurt with improved manufacturability and preservation stability can be provided conveniently and at a low cost.

### [Brief Description of the Drawings]

Fig. 1 shows a syneresis rate of each yogurt.
Fig. 2 shows the results of sensory evaluation of the smoothness of each yogurt.
Fig. 3 shows the results of sensory evaluation of the viscosity sense of each yogurt.

### [Description of Embodiments]

The present invention relates to a production method of yogurt, including a step of adding protein glutaminase and starch to raw milk (hereinafter sometimes to be abbreviated as "the method of the present invention").

While the method of the present invention is characterized by the above-mentioned 2 steps, other steps such as the following conventional ones can be applied.
(1) a preparation step of raw milk
(2) a sterilizing step of raw milk
In the present invention, a step of adding protein glutaminase and a step of adding starch are present. In addition to the above-mentioned, a cooling step and a step of adding other additive may also be included as appropriate.

### Preparation step of raw milk

The raw milk in the present invention may be, for example, milk of mammals such as cow's milk, goat milk etc., defatted milk thereof, homogenized milk, processed milk, concentrates thereof, milk diluted with water, or dry powdered milk suspended and dissolved in water.

The fat content of milk in raw milk in the present invention is not more than 1.5 wt%, preferably not more than 1 wt%, more preferably less than 0.5 wt%. The fat content of milk may be 0, generally not less than 0.1 wt%, preferably not less than 0.3 wt%. The component standard of milkfat in cow's milk in Japan is not less than 0.5% and not more than 1.5% of milkfat in low-fat cow's milk, and less than 0.5% in nonfat cow's milk.

The protein content in raw milk is not more than 5.5 wt%, preferably not more than 4 wt%, more preferably less than 4 wt%, further preferably not more than 3.5 wt%. In consideration of the addition of protein glutaminase, the content is not less than 0.5 wt%, preferably not less than 1.5 wt%, more preferably not less than 2.5 wt%.

As the raw milk in the present invention, commercially available nonfat milk may be used or, for example, milk containing milkfat and protein adjusted to fall within the above-mentioned ranges according to a conventional method of adjusting the concentration of milk protein in consideration of the amount of protein in defatted powdered milk by dissolving the defatted powdered milk and the like in water or the like may be used.

### Step of adding protein glutaminase

The "step of adding protein glutaminase" characterizing the present invention is a step where protein glutaminase is added to raw milk.

As the protein glutaminase in the present invention, a commercially available one or one prepared from a culture medium of a microorganism producing protein glutaminase can be used. As the preparation method thereof, a known method for protein separation and purification (centrifugation, UF concentration, salting out, various chromatographys using ion exchange resin and the like, etc.) can be used. For example, a culture medium is centrifuged to remove bacteria, after which salting out, chromatography and the like are combined to give the object enzyme. When an enzyme is recovered from bacteria, for example, bacteria are disrupted by pressurization treatment, sonication and the like, and separation and purification similar to the above are performed to give the object enzyme. The above-mentioned series of steps (disruption of bacteria, separation, purification) may also be performed after recovering the bacteria in advance from a culture medium by filtration, centrifugation treatment and the like. The enzyme may be powderized by a drying method such as freeze-drying, drying under reduced pressure and the like, during which a suitable excipient or a drying aid may also be used.

The kind of protein glutaminase in the present invention is not particularly limited as long as it directly acts on an amide group of protein to deamidate same without cleavage of peptide bond and crosslinking of protein. Examples of such enzyme include, but are not particularly limited to, protein glutaminases derived from Chryseobacterium, Flavobacterium or Empedobacter disclosed in JP-A-2000-50887, JP-A-2001-218590, WO 2006/075772, commercially available protein glutaminase derived from Chryseobacterium and the like.

The activity of the protein glutaminase to be used in the present invention is measured by the following method.
(1) An aqueous solution (0.1 ml) containing protein glutaminase is added to 0.2 M phosphoric acid buffer (pH 6.5) (1 ml) containing 30 mM Z-Gln-Gly, and the mixture is incubated at 37°C for 10 min and the reaction is discontinued by adding 0.4 M TCA solution (1 ml) . As a blank, a mixture of 0.2 M phosphoric acid buffer (pH 6.5) (1 ml) containing 30 mM Z-Gln-Gly and 0.4 M TCA solution (1 ml) added with an aqueous solution (0.1 ml) containing protein glutaminase is incubated at 37°C for 10 min.
(2) Using ammonia Test Wako (Wako Pure Chemical Industries, Ltd.), the amount of ammonia produced by the reaction of the solution of (1) is measured. The concentration of ammonia in the reaction mixture is determined from the analytical curve showing the relationship between the ammonia concentration and absorbance (630 nm), which was plotted using the ammonia standard solution (ammonium chloride).
(3) The activity of protein glutaminase is calculated from a predetermined formula with the amount of enzyme that produces 1 µmol of ammonia for 1 minute as one unit.

The amount of protein glutaminase to be added is generally not less than 0.05 unit, preferably not less than 0.1 unit, more preferably not less than 0.5 unit, per 1 g in weight of milk protein in raw milk. Similarly, it is generally not more than 100 units, preferably not more than 25 units, more preferably not more than 10 units. Smooth yogurt with low syneresis rate can be obtained when the amount is within the above-mentioned range.

### Step of adding starch

The "step of adding starch" characterizing the present invention is a step where the starch is added to raw milk.

The starch in the present invention is not particularly limited as long as it is removed and purified from plants. Examples thereof include starch derived from potatoes such as white potato, sweet potato and the like, graincereals such as wheat, rice, corn and the like, vegetable, root vegetable, fruits. Of these, starch of potatoes or graincereals is preferable, that of white potato (*bareisho*), rice, wheat or corn is more preferable, that of rice, wheat or corn is further preferable, and that of rice or corn is particularly preferable.

The starch in the present invention is preferably starch free of pregelatinizing treatment (β starch). When unpregelatinized starch is used, the yogurt can be provided to not only healthy individuals but also patients in need of low GI (Glycemic Index) diet.

The average particle size of starch is generally less than 40 µm, preferably not more than 30 µm, more preferably not more than 20 µm, further preferably not more than 15 µm, from the aspects of smoothness and viscosity. From the aspects of mixability when used as a preparation, it is not less than 1 µm, more preferably not less than 2 µm.

The average particle size is measured by a dynamic light scattering measurement method. For example, using dynamic light scattering apparatus Zetasizer Nano ZS (Malvern), 800 µl each of starch diluted 10-fold with MilliQ water is filled as a measurement sample in a vial and the particle size distribution and average particle size are measured by Autosampler (each test fraction n=3).

The amount of starch to be added is generally not less than 0.1 wt%, preferably not less than 0.5 wt%, and more preferably not less than 0.8 wt%, relative to the raw milk. From the aspect of cost, it is not more than 10 wt%, preferably not more than 8 wt%, more preferably not more than 5 wt%.

### Step of adding starter

In the method of the present invention, examples of the starter include lactobacillus and yogurt (starter culture) containing remaining live lactobacillus.

The amount of the starter to be added can be determined by a conventional method according to the kind of the raw milk and starter. For example, the amount of the lactobacillus starter (Chr. Hansen, YC-370) for yogurt to be added is generally 0.0001% - 1%, preferably 0.01% - 0.5%.

In the method of the present invention, the steps of adding protein glutaminase and starch may be performed simultaneously or performed with time difference. In addition, the steps of adding protein glutaminase, starch and starter may be performed simultaneously or performed with time difference.

### Fermentation step

The temperature at which raw milk is fermented by adding starch, protein glutaminase and a starter is 0 - 80°C, preferably 3 - 50°C. The fermentation time is generally 1 - 8 hr, preferably 2 - 6 hr. The pH during fermentation is generally 3.5 - 6.0, and the fermentation is complete when it reaches 4.0 - 5.0.

These conditions can be appropriately modified or adjusted according to the purity of enzyme to be used, the kind and purity of protein and the like.

A specific production method of yogurt in the present invention includes preparing raw milk with fat and protein starting material within the above-mentioned range, adding additive and the like such as sugar, flavor and the like thereto as necessary, and homogenizing the mixture by a homogenizer and the like. Then, after sterilizing and cooling by a conventional method, starch, protein glutaminase and a starter (lactobacillus, yogurt containing remaining live lactobacillus, etc.) are added, filled in a container and fermented, or fermented in a tank and filled in a container to produce yogurt. When sturdy type yogurt is produced as necessary, the obtained yogurt may be disrupted using a sieve (e.g., pore size 500 µm) or a kitchen aid mixer.

The milk fat and protein contents of yogurt produced by the method of the present invention are the same as the milk fat and protein contents of raw milk since they hardly change due to lactic acid fermentation. The content of milk fat and protein in the yogurt is not more than 1.5 wt% and not more than 5.5 wt% for each.

The starch content of the yogurt obtained by the method of the present invention is generally 0.1 - 10 wt%, preferably 0.5 - 5 wt%.

The shape of the yogurt in the present invention is, for example, solid, semi-solid or liquid, preferably solid or semi-solid. Specifically, set yogurt, sturdy yogurt and the like can be mentioned.

As the additive other than starch and protein glutaminase, the starting materials and additives generally used for the production of yogurt can be used. For example, carbohydrate, stabilizer, emulsifier, colorant, flavor adjuster, antioxidant and the like can be mentioned. Where necessary, vitamins, minerals, fruit pulp and fruit juice of strawberry and the like, solid food such as chocolate and the like may be added.

### [Example]

The present invention is further explained in the following by referring to Examples. The sensory evaluation in the Examples was performed by, unless otherwise specified, a well-trained specialized panel that has engaged in food business for not less than 10 years. In the present specification, unless otherwise specified, % shows wt%.

### <Production Example 1>

### Preparation of sturdy yogurt

(1) As the starting materials, nonfat milk (lipid 0.1%, protein 3.5%; Koiwai Dairy Products CO., LTD.), and cow's milk (lipid 0.1%, protein 3.8%; Koiwai Dairy Products CO., LTD.) are used. Each milk is measured by 300 g, placed in a container and heated at 95°C for 3 min.
(2) After heating, each milk is cooled to 50°C in ice water.
(3) A starter (YC-370, Chr. Hansen Japan) (17.7 mg/mL milk) and protein glutaminase (PG) (500U/g, Amano Enzyme Inc.) and various starches at the ratios indicated in Tables 3 and 4 are added to the above-mentioned milk.
(4) The mixture is fermented in an incubator at 44°C for 4 - 5 hr until pH reaches 4.6.
(5) The yogurt obtained by fermentation is cooled to 20°C in ice water.
(6) The yogurt is disrupted (sturdy) by a sieve (sieve-pore size 500 µm) and sufficiently cooled in a refrigerator.

### <Test method 1>

### Measurement of syneresis rate

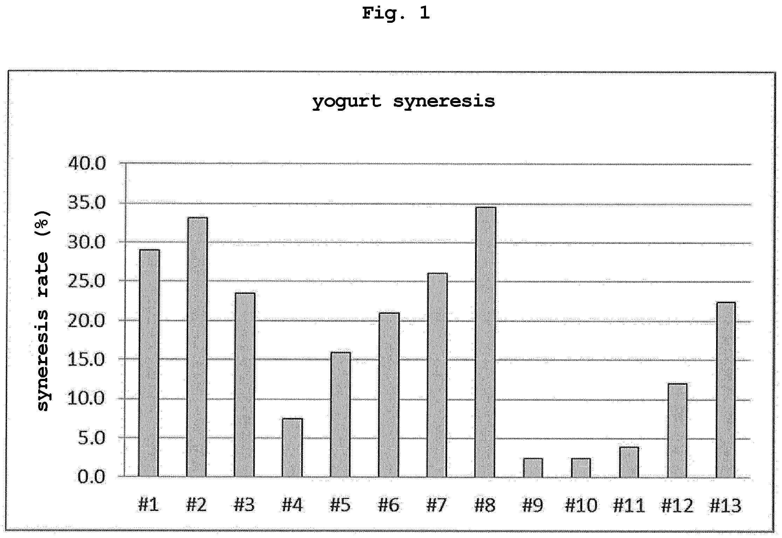

(1) For syneresis measurement, filter paper is placed on a cup, 20 g of yoghurt is placed on the filter paper and water is allowed to be discharged with gravity for 20 minutes.
(2) Water accumulated in the cup is measured and syneresis weight/yogurt initial weight is calculated as the syneresis rate (%). The results are shown in Tables 3 and 4 and Fig. 1.

### <Test method 2>

### Viscosity evaluation

Using a dynamic viscoelasticity measurement apparatus, the viscosity at 24°C was measured. The viscosity at 100 (1/S) when the shear rate was increased to 0⇒100 (1/S) was measured. The results are shown in Tables 3 and 4.

### <Test method 3>

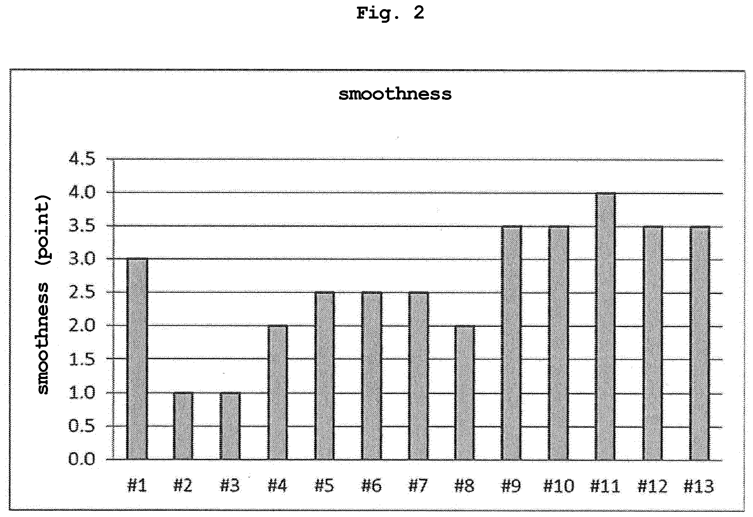

According to the criteria shown in the following Table 1 and with the "smoothness" being a mouthfeel when yogurt eaten is not felt rough and not remaining in the mouth, sensory evaluation was conducted in 7 stages by three expert panels. Average sensory evaluation of each panelist is shown in Tables 3 and 4 and Fig. 2.

**[Table 1]**

| | |
|---|---|
| 6 | very smooth and most preferable |
| 5 | very smooth and preferable |
| 4 | more smooth and preferable |
| 3 | smooth and tolerable |
| 2 | low smoothness |
| 1 | considerably lower smoothness |
| 0 | lowest smoothness |

### <Test method 4>

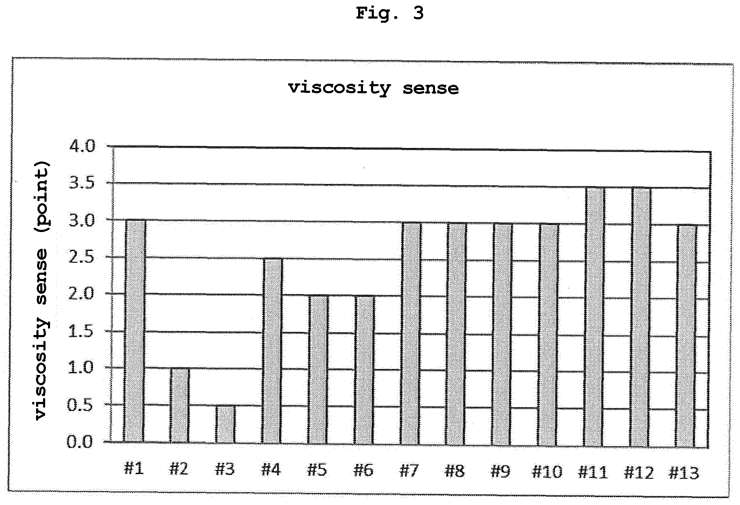

According to the criteria shown in the following Table 2 and with the strength of viscosity sensed from yogurt as "viscosity sense", sensory evaluation was performed in seven stages by three expert panels. Average sensory evaluation of each panelist is shown in Tables 3 and 4 and Fig. 3.

**[Table 2]**

| | |
|---|---|
| 6 | very strong viscosity and most preferable |
| 5 | very high viscosity and preferable |
| 4 | higher viscosity and preferable |
| 3 | tolerable viscosity |
| 2 | low viscosity |
| 1 | very low viscosity |
| 0 | viscosity not felt |

### <Total evaluation>

With the above-mentioned test results, total evaluation was performed according to the following criteria.
⊙: yogurt with high smoothness and viscosity and most preferable
○: yogurt with appropriate smoothness and viscosity and very preferable
Δ: yogurt with appropriate smoothness and viscosity and preferable
×: unpreferable as yogurt

The results are shown in Tables 3 and 4. From the results of #9 - #13 obtained by adding protein glutaminase and each starch and fermenting, it was confirmed that smooth yogurt with strong viscosity sense and low syneresis can be obtained even though it is low-fat and low-protein yogurt.

**[Table 3]**

| No. | #1 | #2 | #3 | #4 | #5 | #6 | #7 | #8 |
|---|---|---|---|---|---|---|---|---|
| protein % | 3.8 | 3.5 | 3.5 | 3.5 | 3.5 | 3.5 | 3.5 | 3.5 |
| lipid % | 0.1 | 0.1 | 0.1 | 0.1 | 0.1 | 0.1 | 0.1 | 0.1 |
| PG (U/g protein) | | | 1.2 | | | | | |
| cornstarch (%) | | | | 1 | | | | |
| glutinous rice starch (%) | | | | | 1 | | | |
| rice starch (%) | | | | | | 1 | | |
| wheat starch (%) | | | | | | | 1 | |
| potato starch (%) | | | | | | | | 1 |
| viscosity (mPa sec) | 71.7 | 57.4 | 40.3 | 75.0 | 54.3 | 49.1 | 88.6 | 56.5 |
| syneresis rate (%) | 28.9 | 33.2 | 23.5 | 7.5 | 16.0 | 21.0 | 26.1 | 34.5 |
| smoothness (point) | 3.0 | 1.0 | 1.0 | 2.0 | 2.5 | 2.5 | 2.5 | 2.0 |
| viscosity sense (point) | 3.0 | 1.0 | 0.5 | 2.5 | 2.0 | 2.0 | 3.0 | 3.0 |
| total evaluation | ○ | × | × | × | × | × | × | × |

**[Table 4]**

| No. | #9 | #10 | #11 | #12 | #13 |
|---|---|---|---|---|---|
| protein % | 3.5 | 3.5 | 3.5 | 3.5 | 3.5 |
| lipid % | 0.1 | 0.1 | 0.1 | 0.1 | 0.1 |
| PG (U/g protein) | 1.2 | 1.2 | 1.2 | 1.2 | 1.2 |
| cornstarch (%) | 1 | | | | |
| glutinous rice starch (%) | | 1 | | | |
| rice starch (%) | | | 1 | | |
| wheat starch (%) | | | | 1 | |
| potato starch (%) | | | | | 1 |
| viscosity (mPa sec) | 47.1 | 56.1 | 61.8 | 74.0 | 60.8 |
| syneresis rate (%) | 2.5 | 2.5 | 4.0 | 12.1 | 22.4 |
| smoothness (point) | 3.5 | 3.5 | 4.0 | 3.5 | 3.5 |
| viscosity sense (point) | 3.0 | 3.0 | 3.5 | 3.5 | 3.0 |
| total evaluation | ⊙ | ⊙ | ⊙ | ○ | Δ |

As various starches, those described in Table 5 were used.

**[Table 5]**

| kind | name | supplier | average particle size |
|---|---|---|---|
| glutinous rice starch | Motiru B | Joetsu Starch Co., Ltd. | 4 - 5 µm |
| rice starch | Finesnow | Joetsu Starch Co., Ltd. | 4 - 5 µm |
| cornstarch | Nisshoku waxy cornstarch MD | Nihon shokuhin kako co., ltd. | 15 µm |
| wheat starch | Kitanoyuki | Kitaguni starch | 16-40 µm |
| potato starch | Nisshoku ginrei | Nihon shokuhin kako co., ltd. | not less than 40 µm |

<Production Example 2>

### Preparation of set yogurt

(1) As the starting material, nonfat milk (lipid 0.1%, protein 3.5%; Koiwai Dairy Products CO., LTD.) is used. The nonfat milk (300 g) is measured, placed in a container and heated at 95°C for 3 min.
(2) After heating, the nonfat milk is cooled to 50°C in ice water.
(3) A starter (YC-370, Chr. Hansen Japan) (17.7 mg/mL nonfat milk), and protein glutaminase (PG) (500U/g, Amano Enzyme Inc.) and rice starch (Finesnow described in Table 5) at the ratios indicated in Table 6 are added to the above-mentioned nonfat milk.
(4) The obtained mixture is divided by 40 g in a cup and fermented in an incubator at 44°C for 4 - 5 hr until pH reaches 4.6.
(5) The yogurt produced by fermentation is cooled in a refrigerator.

The obtained yogurt was subjected to a test similar to the above-mentioned test methods 3 and 4, sensory evaluation was performed in seven stages by three expert panels according to the criteria shown in the above-mentioned Tables 1 and 2. Average sensory evaluation of each panelist is shown in Table 6. With the test results, total evaluation was performed according to the above-mentioned criteria.

**[Table 6]**

| test fraction | without addition | PG 1U/gp | rice starch 1% | rice starch 1% +PG 1 U/gp |
|---|---|---|---|---|
| No. | #1A | #2A | #3A | #4A |
| protein % | 3.5 | 3.5 | 3.5 | 3.5 |
| fat % | 0.1 | 0.1 | 0.1 | 0.1 |
| PG (U/g protein) | | 1.0 | | 1.0 |
| rice starch (%) | | | 1.0 | 1.0 |
| smoothness (point) | 1.0 | 1.0 | 1.5 | 4.0 |
| viscosity sense (point) | 1.0 | 0.5 | 2.0 | 4.0 |
| total evaluation | × | × | × | ⊙ |

As shown in Table 6, the results of #4A obtained by adding protein glutaminase and starch and fermenting them confirm that smooth yogurt with strong viscosity sense is obtained even though it is low-fat and low-protein yogurt.

### [Industrial Applicability]

According to the present invention, smooth low-fat and low-protein yogurt superior in shape retainability can be provided.

## Claims

1. A method of producing yogurt comprising a step of adding protein glutaminase to raw milk and a step of adding starch to raw milk, wherein the raw milk has a fat content of milk of not more than 1.5 wt% and a protein content of not more than 5.5 wt%.

2. The method according to claim 1, wherein the starch has an average particle size of not less than 1 µm and less than 40 µm, the average particle size is measured by dynamic light scattering as defined in the description.

3. The method according to claim 1 or 2, wherein the amount of the starch to be added is not less than 0.1 wt% and not more than 10.0 wt% relative to the raw milk.

4. The method according to any one of claims 1 to 3, wherein the amount of protein glutaminase to be added is not less than 0.05 unit and not more than 100 units per 1 g in weight of the milk protein in the raw milk, the method of how to determine one unit of protein glutaminase activity is defined in the description.

5. The method according to any one of claims 1 to 4, wherein the step of adding protein glutaminase is performed before a fermentation step.

6. The method according to any one of claims 1 to 5, wherein the step of adding starch is performed before the fermentation step.

## Patentansprüche

1. Verfahren zur Herstellung von Joghurt, umfassend einen Schritt der Zugabe von Proteinglutaminase zu Rohmilch und einen Schritt der Zugabe von Stärke zu Rohmilch, wobei die Rohmilch einen Milchfettgehalt von nicht mehr als 1,5 Gew.-% und einen Proteingehalt von nicht mehr als 5,5 Gew.-% aufweist.

2. Verfahren nach Anspruch 1, wobei die Stärke eine durchschnittliche Teilchengröße von nicht weniger als 1 µm und weniger als 40 µm aufweist, wobei die durchschnittliche Teilchengröße durch dynamische Lichtstreuung wie in der Beschreibung definiert gemessen wird.

3. Verfahren nach Anspruch 1 oder 2, wobei die auf die Rohmilch bezogene Menge der zuzusetzenden Stärke nicht weniger als 0,1 Gew.-% und nicht mehr als 10,0 Gew.-% beträgt.

4. Verfahren nach einem der Ansprüche 1 bis 3, wobei die Menge der zuzusetzenden Proteinglutaminase nicht weniger als 0,05 Einheiten und nicht mehr als 100 Einheiten pro 1 g Gewicht des Milchproteins in der Rohmilch beträgt, wobei die Methode zur Bestimmung einer Einheit der Proteinglutaminaseaktivität in der Beschreibung definiert ist.

5. Verfahren nach einem der Ansprüche 1 bis 4, wobei der Schritt der Zugabe von Proteinglutaminase vor einem Fermentationsschritt durchgeführt wird.

6. Verfahren nach einem der Ansprüche 1 bis 5, wobei der Schritt der Zugabe von Stärke vor dem Fermentationsschritt durchgeführt wird.

## Revendications

1. Procédé pour produire du yaourt, comprenant une étape d'addition de protéine glutaminase à du lait cru et une étape d'addition d'amidon à du lait cru, dans lequel le lait cru a une teneur en matières grasses du lait non supérieure à 1,5 % en poids et une teneur en protéines non supérieure à 5,5 % en poids.

2. Procédé selon la revendication 1, dans lequel l'amidon a une granulométrie moyenne non inférieure à 1 µm et inférieure à 40 µm, laquelle granulométrie moyenne est mesurée par diffusion dynamique de la lumière comme défini dans la description.

3. Procédé selon la revendication 1 ou 2, dans lequel la quantité de l'amidon à ajouter n'est pas inférieure à 0,1 % en poids et pas supérieure à 10,0 % en poids par rapport au lait cru.

4. Procédé selon l'une quelconque des revendications 1 à 3, dans lequel la quantité de protéine glutaminase à ajouter n'est pas inférieure à 0,05 unité et pas supérieure à 100 unités par gramme en poids des protéines de lait dans le lait cru, le procédé pour déterminer une unité d'activité de protéine glutaminase étant défini dans la description.

5. Procédé selon l'une quelconque des revendications 1 à 4, dans lequel l'étape d'addition de protéine glutaminase est réalisée avant une étape de fermentation.

6. Procédé selon l'une quelconque des revendications 1 à 5, dans lequel l'étape d'addition d'amidon est réalisée avant l'étape de fermentation.
